# EUROPÄISCHE PATENTSCHRIFT

(11) **EP 2 723 269 B1**
(45) Veröffentlichungstag und Bekanntmachung des Hinweises auf die Patenterteilung: **26.06.2019**
(21) Anmeldenummer: 12743064.3
(22) Anmeldetag: 23.04.2012
(51) Int. Cl.: A61B 34/30

(54) **ROBOTER ZUM HALTEN UND ZUR HANDHABUNG MEDIZINISCHER INSTRUMENTE/GERÄTSCHAFTEN**
ROBOT FOR HOLDING AND FOR HANDLING MEDICAL INSTRUMENTS/EQUIPMENT
ROBOT DESTINÉ À TENIR ET À MANIPULER DES INSTRUMENTS/ÉQUIPEMENTS MÉDICAUX

(30) Priorität: 24.06.2011 DE 102011105748
(43) Veröffentlichungstag der Anmeldung: 30.04.2014
(73) Patentinhaber: Ruprecht-Karls-Universität Heidelberg, 69047 Heidelberg (DE)
(72) Erfinder: MERSCHER, Michael, 63263 Neu-Isenburg (DE); GUNDLING, Ralf, 76709 Kronau (DE); SCHWARZ, Markus, 82067 Ebenhausen (DE); HESSER, Juergen, 69118 Heidelberg (DE); POTT, Peter, P., 68309 Mannheim (DE); BOESECKE, Robert, 69123 Heidelberg (DE); BRODERSEN, Jens, 68549 Ilvesheim (DE); VIEIRA, Vitor, 69115 Heidelberg (DE); LISIAK, Eugen, 68239 Mannheim (DE); NGUYEN, Am Tuong, 68161 Mannheim (DE)
(74) Vertreter: Patent- und Rechtsanwälte Ullrich & Naumann
(86) Internationale Anmeldenummer: PCT/DE2012/200029
(87) Internationale Veröffentlichungsnummer: WO 2012/175081

(56) Entgegenhaltungen:
- DE-A1-102006 007 858
- US-A1- 2003 181 800
- US-A1- 2009 248 037
- US-A1- 2009 248 039

## Beschreibung

Die Erfindung betrifft einen Roboter zum Halten und zur Handhabung medizinischer Instrumente/Gerätschaften, insbesondere Retraktoren, vorzugsweise zur Anwendung bei orthopädischen Operationen.

Bei den in Rede stehenden Halte- und Handhabungsaufgaben kann es sich im Konkreten um das Halten von Retraktoren während eines chirurgischen Eingriffs handeln. Retraktoren sind chirurgische Halteinstrumente, die zum Offenhalten eines Operationsfeldes eingesetzt werden.

Bisher werden Halte- und Handhabungsaufgaben meist von medizinischem Personal ausgeführt. Die zum Offenhalten des Operationsfeldes eingesetzten Retraktoren müssen vom Assistenten dabei derart gehalten werden, dass der Operateur bestmögliche Sicht und Bewegungsfreiheit zum Durchführen des Eingriffs hat. Dabei ist von besonderer Bedeutung, dass das Gewebe durch die Retraktoren nicht zu stark auf Zug belastet wird, um Verletzungen des Patienten zu vermeiden. Die Retraktoren müssen daher mitunter vom Assistenten nachgeführt werden, wenn äußere Störungen bzw. Kräfte auftreten. Das Halten der Retraktoren ist eine kraft- und zeitintensive Aufgabe, die physisch anstrengend und ermüdend ist. Insbesondere bei orthopädischen Operationen, wie bspw. Hüft-Totalendoprothetischen-Eingriffen (Hüft-TEP), wirken große Kräfte, so dass die Halteaufgabe für den Assistenten sehr anstrengend ist. Da die Halte- und Handhabungsaufgaben sehr häufig von einem approbierten Arzt ausgeführt werden, entstehen durch das Halten der Retraktoren hohe Kosten.

Des Weiteren sind aus der Praxis Vorrichtungen bekannt, welche bspw. am Operationstisch fixiert werden und an denen ein Halteinstrument bzw. Retraktor montierbar ist. Zur Ausgleichung von externen Störungen, wie sie insbesondere bei orthopädischen Operationen auftreten können, werden druckluftgedämpfte Systeme eingesetzt. Hierbei ist jedoch problematisch, dass die Retraktoren starr am Halterahmen fixiert sind, so dass sie nicht aktiv nachjustierbar sind. Dies führt dazu, dass sich die Retraktoren unbemerkt lockern können oder im Bedarfsfalle nicht nachgeben, so dass es u. a. zu Behinderungen im Operationsverlauf oder gar zu Verletzungen von Gewebe und Weichteilen des Patienten kommen kann. Weiterhin problematisch ist die zeit- und personalintensive Montage des starren Halterahmens vor dem Beginn der eigentlichen Operation, wobei der Halterahmen steril gehalten werden muss, wodurch abermals Kosten entstehen.

Schließlich sind Roboter zur Anwendung bei chirurgischen Eingriffen seit vielen Jahren bekannt. Lediglich beispielhaft sei hierbei auf die DE 102 39 673 A1 hingewiesen, die eine Vorrichtung zur Bearbeitung von Teilen, insbesondere von Knochen, Organen, etc. des menschlichen und tierischen Körpers zeigt. Robotersysteme dieser Art eignen sich jedoch nicht zum Halten und zur Handhabung medizinischer Instrumente/Gerätschaften, wie z.B. Retraktoren, da keine Anpassung des Haltevorgangs an externe Störungen vorgesehen ist. Die in Rede stehenden Robotersysteme arbeiten vielmehr ferngesteuert, wobei der Roboter die Handbewegungen des Chirurgen nachvollzieht.

Das Dokument DE-A-10 2006 007 858 zeigt einen Chirurgieroboter, welcher ein Kraftmessmodul aufweist, damit der Roboter bei Überschreitung vorgegebener Kräfte abgeschaltet wird, um das Gewebe nicht zu verletzen. Der vorliegenden Erfindung liegt daher die Aufgabe zugrunde, einen Roboter zum Halten und zur Handhabung medizinischer Instrumente, insbesondere Retraktoren, derart auszugestalten, dass bei minimalem Personalaufwand kraft- und zeitintensive Halte- und die Handhabungsaufgaben, die mitunter externen Störungen unterliegen, sicher ausführbar sind.

Erfindungsgemäß wird die voranstehende Aufgabe durch die Merkmale des Patentanspruchs 1 gelöst. Danach ist ein Roboter zum Halten und zur Handhabung medizinischer Instrumente/Gerätschaften mit einem Manipulator und einem vom Manipulator getragenen Endeffektor zum Ergreifen/Ankoppeln des jeweiligen Instruments angegeben, wobei Mittel zum Erfassen äußerer Parameter betreffend die Haltesituation vorgesehen sind und wobei aufgrund der ermittelten Parameter und ggf. unter Nutzung weiterer vorgebbarer Parameter die Halte-/Handhabungsfunktion des Roboters definierbar ist.

In erfindungsgemäßer Weise ist erkannt worden, dass sich ein Roboter zur Übernahme von Halte- und Handhabungsaufgaben in idealer Weise verwenden lässt. Dazu weist der Roboter einen Manipulator und einen vom Manipulator getragenen Endeffektor zum Ergreifen/Ankoppeln des jeweiligen Instruments auf. In erfindungsgemäßer Weise ist des Weiteren erkannt worden, dass eine aktive Halte-/Handhabungsfunktion des Roboters in überraschend einfacher Weise definierbar ist, wenn Mittel zum Erfassen äußerer Parameter betreffend die Haltesituation vorgesehen sind. Hierbei kann es sich bspw. um auftretende Kräfte handeln, die vom Roboter erfassbar sind und anhand derer die Halte-/Handhabungsfunktion des Roboters definierbar ist. Schließlich ist erkannt worden, dass die Halte-/Handhabungsfunktion des Roboters unter Nutzung weiterer vorgebbarer Parameter, die bspw. der Operateur vor oder während des Eingriffs vorgibt, weiter definierbar ist. Das Wissen und die Erfahrung des Operateurs sind daher der Halte-/Handhabungsfunktion des Roboters zugrunde gelegt. Durch diese konstruktiven Merkmale ist somit ein Roboter angegeben, der zeit- und kraftintensive Halte- und Handhabungsaufgaben sicher erfüllt, so dass beispielsweise Retraktoren nicht mehr von medizinischem Personal gehalten werden müssen.

In vorteilhafter Weise sind die Mittel zum Erfassen äußerer Parameter als Sensoren zum Erfassen von am Manipulator und/oder am Endeffektor angreifender äußerer Kräfte ausgeführt. Die äußeren Kräfte können in eine entsprechende Bewegung des Manipulators und/oder des Endeffektors umsetzbar sein, so dass der Endeffektor in eine Halte-/Handhabungsposition an das Instrument verbringbar ist. Durch dieses kraftfreie Führen lässt sich der Roboter bzw. der Endeffektor vom Operateur an eine gewünschte Stelle im Raum führen. Hierbei ist denkbar, dass vom Operateur oder dem medizinischen Personal das Instrument bzw. der Retraktor bereits in das Operationsfeld eingeführt und den Anforderungen des Operateurs entsprechend positioniert ist. An dieser Stelle sei darauf hingewiesen, dass der Operateur somit insbesondere die Kraft, die der Retraktor auf das Gewebe ausübt, vorgibt. Der Roboter kann nunmehr an das Instrument herangeführt werden und das bereits exakt positionierte Instrument vom Operateur übernehmen. Durch diese konstruktive Maßnahme ist es daher möglich, dass der Roboter eine vom Operateur begonnene und somit exakt definierte Halteaufgabe übernimmt. Des Weiteren ist denkbar, dass der Operateur bei Bedarf, bspw. am Ende des Eingriffs, das Instrument wieder vom Roboter übernimmt. Der Roboter würde somit eine Halteaufgabe, die der Operateur beginnt und auch wieder beendet, zwischenzeitlich übernehmen. Aufwendige globale Tracking-/Positionssysteme zum Heranführen des Roboters an das Instrument bzw. das Operationsfeld entfallen.

Hinsichtlich einer exakten Definierbarkeit der Halte-/Handhabungsfunktion des Roboters sind bei oder nach dem Ergreifen/Ankoppeln des Instruments an den Endeffektor äußere Parameter erfassbar. Als äußere Parameter sind beispielsweise die am Endeffektor auftretenden Kräfte und/oder die Orientierung des Endeffektors im Raum und/oder die Arbeitsraumgrenzen des Endeffektors erfassbar. Durch diese konstruktive Maßnahme wird eine Kalibrierung des Roboters zum Instrument bzw. zum Retraktor auf indirektem Wege erreicht. Die Sensoren erfassen bei oder nach dem Ergreifen/Ankoppeln des Instruments an den Endeffektor beispielsweise solange den Kraftanstieg, bis kein signifikanter Kraftanstieg mehr registrierbar ist. Des Weiteren ist denkbar, durch die Registrierung der Orientierung des Endeffektors im Raum, den Roboter relativ zum Instrument bzw. Retraktor zu kalibrieren. Somit entfällt ein zeitintensives Kalibrieren des Roboters vor dem operativen Einsatz.

In Bezug auf eine aktive Regelung der Halte-/Handhabungsfunktion ist es von besonderem Vorteil, wenn aus den äußeren Parametern und ggf. aus den vorgebbaren Parametern beispielsweise durch eine Kraft- und/oder Impedanzregelung oder durch eine hybride Kraft- und Positionsregelung die für die Halte-/Handhabungsfunktion notwendigen Regelparameter bestimmbar sind.

Zur Eingabe der vorgebbaren Parameter, wie z.B. der Dämpfungseigenschaften des viskoelastischen Gewebes, kann eine Eingabevorrichtung vorgesehen sein. Die Eingabevorrichtung kann als Tastatur oder Touch-Screen ausgeführt sein. Des Weiteren ist denkbar, dass die Eingabevorrichtung zur Aktivierung und Deaktivierung der Halte-/Handhabungsfunktion, vorzugsweise als Fußschalter, ausgeführt ist.

Um eine aktive Halte-/Handhabungsfunktion des Roboters zu gewährleisten, können die Mittel zum Erfassen äußerer Parameter als Sensoren zum Erfassen der während der Haltesituation zwischen dem Instrument und dem gehaltenen Gewebe auftretenden Kräfte ausgeführt sein. Durch diese konstruktiven Merkmale kann bei Überschreitung eines vorgegebenen Kraftwertes der Endeffektor automatisch in Richtung der auftretenden Kraft nachführbar sein. Der Operateur kann bspw. vor Beginn der Operation über eine Eingabevorrichtung maximale Kraftparameter definieren, so dass bei deren Überschreitung der Endeffektor automatisch in Richtung der auftretenden Kraft nachführbar ist. Des Weiteren ist denkbar, dass ein akustisches und/oder optisches Signal ausgelöst wird, wenn der Messwert den vorgegebenen Kraftwert überschreitet. Die Gefahr von Behinderungen im Operationsbetrieb, die bspw. durch starre Halterahmen hervorgerufen werden können, ist erheblich minimiert, da der Roboter eine aktive Halte-/Handhabungsfunktion am Instrument ausführt.

In Bezug auf eine möglichst einfache Konstruktion, die den speziellen Anforderungen einer medizinischen Operationseinrichtung entspricht, ist der Endeffektor vorteilhafterweise aus mehreren Modulen aufgebaut. Die Module sind vorzugsweise über Kupplungselemente miteinander verbunden. Die Module können dabei lösbar miteinander verbunden sein, um die einzelnen Komponenten des Endeffektors in kurzer Zeit zu demontieren und zu sterilisieren.

Zum Ergreifen bzw. Ankoppeln des Instruments an den Endeffektor kann dieser ein Greifermodul aufweisen. Das Greifermodul kann bspw. als Einzelgreifer ausgeführt sein. Um den Roboter an verschiedene genormte oder auch nicht genormte medizinische Instrumente/Gerätschaften anzupassen, kann das Greifermodul auswechselbar ausgeführt sein. Je nach Operation und dazu benötigtem medizinischen Instrument, ist das Greifermodul auszuwählen und entsprechend am Endeffektor zu montieren. Zur Sicherung gegen ein unerwartetes Lösen der Instrumente/Gerätschaften vom Greifermodul, kann dieses ein Wirksystem und/oder ein kinematisches System aufweisen. In besonders vorteilhafter Weise ist das kinematische System derart ausgebildet, dass es unabhängig von einer externen Energieversorgung ist. Dadurch kann das kinematische System jederzeit durch den Assistenten/Operateur geöffnet oder geschlossen werden. Insbesondere bei einem Ausfall des Roboters - bspw. bei einem "Einfrieren" - kann der Operateur das kinematische System öffnen und das Halteinstrument aus dem Endeffektor übernehmen. Aufgrund der modularen Bauweise des Endeffektors kann das Greifermodul danach demontiert werden, so dass dem Operateur ein ausreichender Arbeitsraum zur Verfügung steht.

Die zur Erfassung der äußeren Parameter benötigten Sensoren können in besonders vorteilhafter Weise in einem Sensorikmodul des Endeffektors untergebracht sein. Das Sensorikmodul kann bspw. über eine mechanische Schnittstelle direkt am Roboterflansch des Manipulators montiert sein. Des Weiteren ist denkbar, dass das Sensorikmodul Sensoren zur Erfassung von inneren Parametern aufweist, so dass bspw. detektierbar ist, welches Greifermodul am Endeffektor montiert ist, so dass die entsprechenden Parameter zur Steuerung des Roboters geladen werden.

Da der Roboter unter sterilen Bedingungen einsetzbar sein muss, kann in besonders vorteilhafter Weise am Endeffektor ein Isolationsmodul zum Trennen eines sterilen Bereichs von einem unsterilen Bereich des Endeffektors vorgesehen sein. Das Isolationsmodul kann bspw. zwischen Sensorikmodul und Greifermodul angeordnet sein, so dass das Sensorikmodul im unsterilen Bereich des Endeffektors liegt. In besonders vorteilhafter Weise ist der unsterile Bereich des Endeffektors mit einer auswechselbaren sterilen Abdeckung, bspw. einer Folie, versehen. Im Konkreten kann die Folie am Isolationsmodul lösbar festgelegt sein und sich über das Sensorikmodul hinweg über den Manipulator erstrecken. Die Folie kann somit vor jeder Operation ausgewechselt werden, so dass der unsterile Bereich des Endeffektors bzw. Manipulators stets abgedeckt ist.

Zur weiteren Minimierung der Vorbereitungszeit kann ein Positionserfassungssystem vorgesehen sein, so dass der Manipulator anhand der erfassten Positionsdaten automatisch in eine optimale Ausgangsposition zum Führen in die Halte-/Handhabungsposition verbringbar ist. Das kraftfreie Führen des Manipulators bzw. Endeffektors an das Instrument muss dann bspw. nur noch geradlinig erfolgen, wodurch die Übergabedauer signifikant reduziert ist.

In weiter vorteilhafter Weise können mehrere Manipulatoren mit einem vom Manipulator getragenen Endeffektor vorgesehen sein, so dass mehrere oder sämtliche zum Offenhalten des Operationsfeldes benötigten Instrumente/Gerätschaften von dem Roboter handhabbar sind.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Darstellung ein Ausführungsbeispiel eines erfindungsgemäßen Roboters,
- Fig. 2: in einer schematischen Darstellung ein Ausführungsbeispiel eines erfindungsgemäßen Endeffektors und
- Fig. 3: ein Flussdiagramm zur schematischen Darstellung eines Ausführungsbeispiels der Systemarchitektur eines erfindungsgemäßen Roboters.

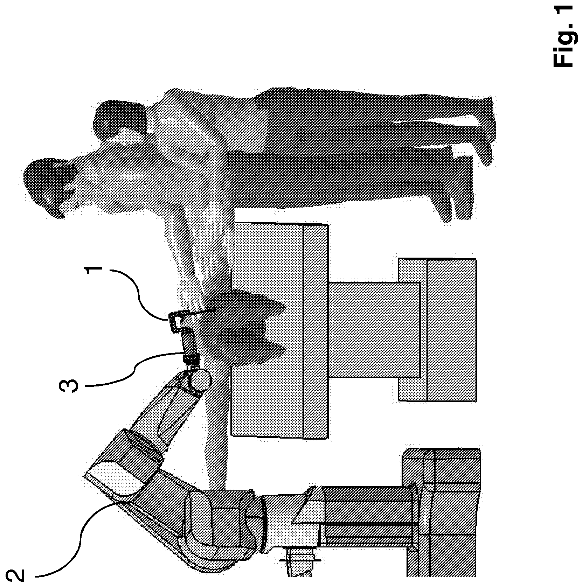

Fig. 1 zeigt in einer schematischen Darstellung einen erfindungsgemäßen Roboter zum Halten eines Retraktors 1 während eines chirurgischen Eingriffs. Der Roboter weist einen von einem Manipulator 2 getragenen Endeffektor 3 auf. Der Endeffektor 3 dient zum Ergreifen und Halten des Retraktors 1. Da der Roboter die Haltefunktion selbstständig aktiv ausführt, kann der Roboter auf äußere Störungen reagieren und den Retraktor beispielsweise nachführen. Die Gefahr von Störungen des Operationsbetriebs wird dadurch auf ein Minimum reduziert.

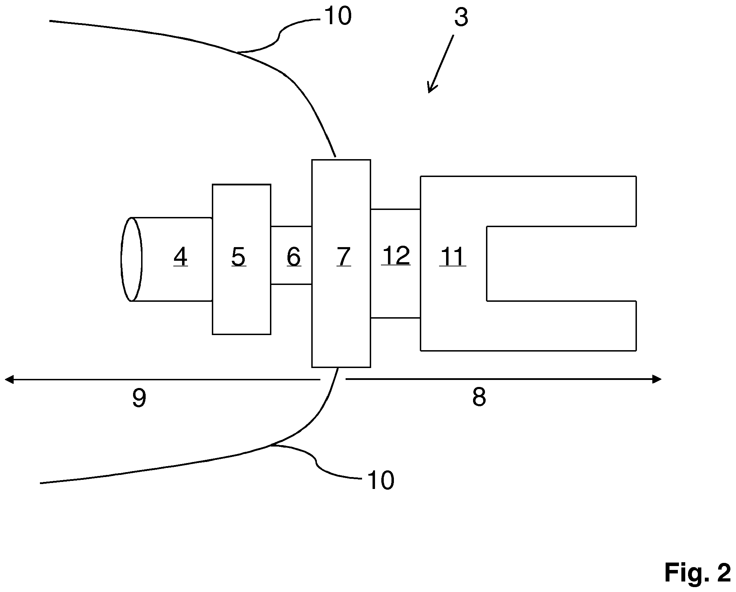

In Fig. 2 ist in einer schematischen Darstellung ein Ausführungsbeispiel eines erfindungsgemäßen Endeffektors 3 dargestellt. Der Endeffektor 3 ist am Roboterflansch 4 des Manipulators festgelegt. Zur Vereinfachung ist in Fig. 2 auf die Darstellung des Manipulators verzichtet. Der Endeffektor 3 weist ein Sensorikmodul 5 auf. In dem Sensorikmodul 5 sind Sensoren zur Erfassung innerer und äußerer Parameter vorgesehen. Am Sensorikmodul 5 ist über ein erstes Kupplungselement 6 das Isolationsmodul 7 festgelegt. Das Isolationsmodul 7 dient zur Unterteilung des Endeffektors 3 in einen sterilen Bereich 8 und einen unsterilen Bereich 9. Dazu weist das Isolationsmodul 7 eine sterile Abdeckung 10 auf, die bspw. als Folie ausgeführt ist. Der Bereich 9 unterhalb der sterilen Abdeckung 10, d.h. der Manipulator 2 samt Roboterflansch 4 und Sensorikmodul 5, gilt als unsteril.

Im sterilen Bereich 8 oberhalb der Abdeckung 10 ist das Greifermodul 11 durch ein zweites Kupplungselement 12 am Isolationsmodul 7 festgelegt. Das Greifermodul 11 ist hier als Einzelgreifer ausgeführt und dient zum Ergreifen/Ankoppeln des Instruments. Die Sensoren im Sensorikmodul 5 erfassen als inneren Parameter welches Greifermodul 11 am Isolationsmodul 7 installiert ist, um die entsprechenden Parameter zur Steuerung des Roboters zu laden. Da sich das Isolationsmodul 7, das zweite Kupplungselement 12 und das Greifermodul 11 im sterilen Bereich 8 befinden, müssen diese sterilisierbar ausgeführt sein.

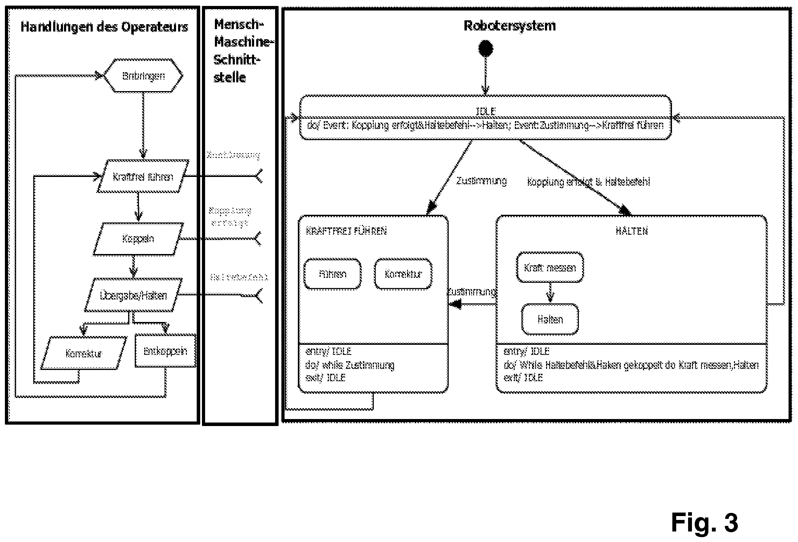

In Fig. 3 ist ein Flussdiagramm zur schematischen Darstellung eines Ausführungsbeispiels der Systemarchitektur eines erfindungsgemäßen Roboters gezeigt. Über die Mensch-Maschinen-Schnittstelle ist der Roboter anhand der Handlungen des Operateurs in seinen Zuständen steuerbar. Hierzu gehören die Zustimmung zum kraftfreien Führen, das Anbringen/Ankoppeln eines Retraktors bzw. Halteinstruments sowie der Befehl zum Ausführen der Haltefunktion. Der Befehl zum Ausführen der Haltefunktion wird über ein Eingabegerät, bspw. über einen Fußschalter, gegeben.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Patentansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Retraktor
- 2: Manipulator
- 3: Endeffektor
- 4: Roboterflansch
- 5: Sensorikmodul
- 6: erstes Kupplungselement
- 7: Isolationsmodul
- 8: steriler Bereich
- 9: unsteriler Bereich
- 10: sterile Abdeckung
- 11: Greifermodul
- 12: zweites Kupplungselement

## Patentansprüche

1. Roboter zum Halten und zur Handhabung medizinischer Instrumente/Gerätschaften (1), insbesondere Retraktoren, vorzugsweise zur Anwendung bei orthopädischen Operationen, mit einem Manipulator (2) und einem vom Manipulator (2) getragenen Endeffektor (3) zum Ergreifen/Ankoppeln des jeweiligen Instruments (1), wobei Mittel zum Erfassen äußerer Parameter betreffend die Haltesituation vorgesehen sind und wobei aufgrund der ermittelten Parameter und ggf. unter Nutzung weiterer vorgebbarer Parameter die Halte-/Handhabungsfunktion des Roboters definierbar ist,
**dadurch gekennzeichnet, dass** am Endeffektor (3) ein Isolationsmodul (7) zum Trennen eines sterilen Bereichs (8) von einem unsterilen Bereich (9) des Endeffektors (3) angeordnet ist, dass das Isolationsmodul (7) zwischen einem Sensorikmodul (5) und einem Greifermodul (11) angeordnet ist, so dass das Sensorikmodul (5) in dem unsterilen Bereich liegt und dass die Mittel zum Erfassen äußerer Parameter als Sensoren zum Erfassen der während der Haltesituation zwischen dem Instrument (1) und dem gehaltenen Gewebe auftretenden Kräfte ausgeführt und bei der Überschreitung eines vorgegebenen Kraftwertes der Endeffektor (3) automatisch in Richtung der auftretenden Kraft nachgeführt wird.

2. Roboter nach Anspruch 1, **dadurch gekennzeichnet, dass** die Mittel zum Erfassen äußerer Parameter als Sensoren zum Erfassen von am Manipulator (2) und/oder am Endeffektor (3) angreifender äußerer Kräfte ausgeführt sind, wobei die äußeren Kräfte in eine entsprechende Bewegung des Manipulators (2) und/oder des Endeffektors (3) umsetzbar sind, so dass der Endeffektor (3) in eine Halte-/Handhabungsposition an das Instrument (1) verbringbar ist.

3. Roboter nach Anspruch 1 oder 2, **dadurch gekennzeichnet, dass** bei oder nach dem Ergreifen/Ankoppeln des Instruments (1) an den Endeffektor (3) als äußere Parameter die am Endeffektor (3) auftretenden Kräfte und/oder die Orientierung des Endeffektors (3) im Raum und/oder die Arbeitsraumgrenzen des Endeffektors (3) erfassbar sind.

4. Roboter nach einem der Ansprüche 1 bis 3 **dadurch gekennzeichnet, dass** aus den äußeren Parametern und ggf. aus den vorgebbaren Parametern durch eine Kraft- und/oder Impedanzregelung oder durch eine hybride Kraft- und Positionsregelung für die Halte-/Handhabungsfunktion notwendige Regelparameter bestimmbar sind.

5. Roboter nach einem der Ansprüche 1 bis 4, **dadurch gekennzeichnet, dass** eine Eingabevorrichtung zur Aktivierung und Deaktivierung der Halte-/Handhabungsfunktion und/oder zur Eingabe der vorgebbaren Parameter vorgesehen ist.

6. Roboter nach einem der Ansprüche 1 bis 5, **dadurch gekennzeichnet, dass** der Endeffektor (3) aus mehreren Modulen (5, 7, 11) aufgebaut ist, wobei
die Module (5, 7, 11) über Kupplungselemente (6, 12), vorzugsweise lösbar, miteinander verbunden sein können.

7. Roboter nach Anspruch 6, **dadurch gekennzeichnet, dass** das Greifermodul (11) als Einzelgreifer ausgeführte ist, wobei
das Greifermodul (11) auswechselbar ausgeführt sein kann.

8. Roboter nach Anspruch 7, **dadurch gekennzeichnet, dass** das Greifermodul (11) ein Wirksystem und/oder ein kinematisches System aufweist.

9. Roboter nach Anspruch 8, **dadurch gekennzeichnet, dass** das kinematische System unabhängig von einer externen Energieversorgung ist, so dass auch bei einem Ausfall des Roboters das medizinische Instrument sicher vom Greifermodul entfernbar ist.

10. Roboter nach einem der Ansprüche 6 bis 9, **dadurch gekennzeichnet, dass** das Sensorikmodul (5) die Sensoren zum Erfassen der äußeren Parametern umfasst.

11. Roboter nach Anspruch 10, **dadurch gekennzeichnet, dass** der unsterile Bereich (9) des Endeffektors (3) mit einer sterilen Abdeckung (10), vorzugsweise einer Folie, überdeckt ist.

12. Roboter nach einem der Ansprüche 1 bis 11, **dadurch gekennzeichnet, dass** ein Positionserfassungssystem vorgesehen ist, so dass der Manipulator (2) anhand der erfassten Positionsdaten automatisch in eine optimale Ausgangsposition zum Führen in die Halte-/Handhabungsposition verbringbar ist.

13. Vorrichtung nach einem der Ansprüche 1 bis 12, **dadurch gekennzeichnet, dass** mehrere Manipulatoren (2) vorgesehen sind.

## Claims

1. Robot for retaining and handling medical instruments/apparatuses (1), in particular retractors, preferably for use in orthopaedic operations, having a manipulator (2) and an end effector (3) which is carried by the manipulator (2) for gripping/coupling the respective instrument (1), wherein means for detecting external parameters relating to the retention situation are provided and wherein, as a result of the established parameters and where applicable using additional parameters which can be predetermined, the retention/handling function of the robot can be defined,
**characterised in that** on the end effector (3) an isolation module (7) for separating a sterile region (8) from a non-sterile region (9) of the end effector (3) is arranged, **in that** the isolation module (7) is arranged between a sensor module (5) and a gripper module (11) so that the sensor module (5) is located in the non-sterile region, and **in that** the means for detecting external parameters are constructed as sensors for detecting the forces which occur during the retention situation between the instrument (1) and the retained tissue and, when a predetermined force value is exceeded, the end effector (3) is automatically tracked in the direction of the force which occurs.

2. Robot according to claim 1, **characterised in that** the means for detecting external parameters are constructed as sensors for detecting external forces which act on the manipulator (2) and/or on the end effector (3), wherein the external forces can be converted into a corresponding movement of the manipulator (2) and/or the end effector (3) so that the end effector (3) can be moved into a retention/handling position on the instrument (1).

3. Robot according to claim 1 or 2, **characterised in that**, during or after the gripping/coupling of the instrument (1) on the end effector (3), the forces which occur on the end effecter (3) and/or the orientation of the end effector (3) in space and/or the operating space limits of the end effector (3) can be detected as external parameters.

4. Robot according to any one of claims 1 to 3, **characterised in that**, from the external parameters and where applicable from the predeterminable parameters, control parameters necessary for the retention/handling function can be determined as a result of a force and/or impedance regulation or as a result of a hybrid force and positioning regulation.

5. Robot according to any one of claims 1 to 4, **characterised in that** an input device is provided for activating and deactivating the retention/handling function and/or for inputting the predeterminable parameters.

6. Robot according to any one of claims 1 to 5, **characterised in that** the end effector (3) is constructed from several modules (5, 7, 11), wherein the modules (5, 7, 11) can be connected to each other, preferably in a releasable manner, by means of coupling elements (6, 12).

7. Robot according to claim 6, **characterised in that** the gripper module (11) is constructed as a single gripper, wherein the gripper module (11) may be constructed so as to be able to be replaced.

8. Robot according to claim 7, **characterised in that** the gripper module (11) has an effector system and/or a kinematic system.

9. Robot according to claim 8, **characterised in that** the kinematic system is independent of an external energy supply so that, even in the event of a failure of the robot, the medical instrument can be safely removed from the gripper module.

10. Robot according to any one of claims 6 to 9, **characterised in that** the sensor module (5) comprises the sensors for detecting the external parameters.

11. Robot according to claim 10, **characterised in that** the non-sterile region (9) of the end effector (3) is covered with a sterile cover (10), preferably a film.

12. Robot according to any one of claims 1 to 11, **characterised in that** a position detection system is provided so that the manipulator (2) can be moved using the detected position data automatically into an optimum starting position for guiding into the retention/handling position.

13. Device according to any one of claims 1 to 12, **characterised in that** a plurality of manipulators (2) are provided.

## Revendications

1. Robot pour le maintien et la manipulation d'instruments/appareillages médicaux (1), plus particulièrement de rétracteurs, de préférence destiné à être utilisé dans des opérations orthopédiques, avec un manipulateur (2) et un effecteur d'extrémité (3) supporté par le manipulateur (2), pour la préhension/le couplage de l'instrument (1) correspondant, des moyens de mesure de paramètres extérieurs concernant la situation de maintien étant prévus et la fonction de maintien/manipulation du robot pouvant être définie sur la base des paramètres déterminés et, le cas échéant, d'autres paramètres prédéterminés,
**caractérisé en ce que**, sur l'effecteur d'extrémité (3) est disposé un module d'isolation (7) pour la séparation d'une zone stérile (8) d'une zone non stérile (9) de l'effecteur d'extrémité (3), **en ce que** le module d'isolation (7) est disposé entre un module de capteur (5) et un module de préhension (11), de façon à ce que le module de capteur (5) se trouve dans la zone non stérile et à ce que les moyens de mesure de paramètres extérieurs soient conçus comme des capteurs pour la mesure des forces exercées pendant la situation de maintien entre l'instrument (1) et le tissu maintenu et soient suivis lors du dépassement d'une valeur de force prédéterminée de l'effecteur d'extrémité (3) automatiquement en direction de la force exercée.

2. Robot selon la revendication 1, **caractérisé en ce que** les moyens de mesure des paramètres extérieurs sont conçus comme des capteurs pour la mesure des forces extérieures appliquées sur le manipulateur (2) et/ou sur l'effecteur d'extrémité (3), les forces extérieures pouvant être appliquées dans un déplacement correspondant du manipulateur (2) et/ou de l'effecteur d'extrémité (3) de façon à ce que l'effecteur d'extrémité (3) puisse être amené dans une position de maintien/manipulation sur l'instrument (1).

3. Robot selon la revendication 1 ou 2, **caractérisé en ce que**, lors de ou après la préhension/le couplage de l'instrument (1) au niveau de l'effecteur d'extrémité (3), en tant que paramètres extérieures, les forces extérieures appliquées à l'effecteur d'extrémité (3) et/ou l'orientation de l'effecteur d'extrémité (3) dans l'espace et/ou les limites de l'espace de travail de l'effecteur d'extrémité (3) peuvent être mesurées.

4. Robot selon l'une des revendications 1 à 3, **caractérisé en ce que**, à partir des paramètres extérieurs et, le cas échéant, à partir des paramètres prédéfinis, une régulation de force et/ou d'impédance ou une régulation de force et de position hybride permet de déterminer les paramètres de régulation nécessaire pour la fonction de maintien/manipulation.

5. Robot selon l'une des revendications 1 à 4, **caractérisé en ce qu'**un dispositif d'entrée est prévu pour l'activation et la désactivation de la fonction de maintien/manipulation et/ou pour l'entrée des paramètres prédéterminés.

6. Robot selon l'une des revendications 1 à 5, **caractérisé en ce que** l'effecteur d'extrémité (3) est constitué de plusieurs modules (5, 7, 11), les modules (5, 7, 11) pouvant être reliés entre eux, de préférence de manière amovible, par l'intermédiaire d'éléments de couplage (6, 12).

7. Robot selon la revendication 6, **caractérisé en ce que** le module de préhension (11) est conçu comme une pince simple, le module de préhension (11) pouvant être réalisé de manière interchangeable.

8. Robot selon la revendication 7, **caractérisé en ce que** le module de préhension (11) comprend un système actif et/ou un système cinématique.

9. Robot selon la revendication 8, **caractérisé en ce que** le système cinématique est indépendant d'une alimentation en énergie externe, de façon à ce que, même dans le cas d'une panne du robot, l'instrument médical peut être retiré du module de préhension en toute sécurité.

10. Robot selon l'une des revendications 6 à 9, **caractérisé en ce que** le module de capteur (5) comprend les capteurs pour la mesure des paramètres extérieurs.

11. Robot selon la revendication 10, **caractérisé en ce que** la zone non stérile (9) de l'effecteur d'extrémité (3) est recouverte d'un couvercle stérile (10), de préférence un film.

12. Robot selon l'une des revendications 1 à 11, **caractérisé en ce qu'**un système de mesure de position est prévu, de façon à ce que le manipulateur (2) puisse être amené automatiquement, à l'aide des données de position mesurées, dans une position initiale optimale pour le guidage vers la position de maintien/manipulation.

13. Dispositif selon l'une des revendications 1 à 12, **caractérisé en ce que** plusieurs manipulateurs (2) sont prévus.
